(19) **Europäisches Patentamt**
**European Patent Office**
**Office européen des brevets**

(11) **EP 4 548 914 A1**

(12) **EUROPEAN PATENT APPLICATION**

(43) Date of publication:
07.05.2025 Bulletin 2025/19

(21) Application number: 24210068.3

(22) Date of filing: 31.10.2024

(51) International Patent Classification (IPC):
*A61K 9/20* (2006.01)    *A61K 31/4985* (2006.01)

(52) Cooperative Patent Classification (CPC):
**A61K 9/20; A61K 31/4985**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 02.11.2023 JP 2023188340

(71) Applicant: **Sawai Pharmaceutical Co., Ltd.**
**Yodogawa-ku**
**Osaka-shi**
**Osaka 532-0003 (JP)**

(72) Inventors:
• **YOSHIHARA, Naoki**
**Osaka City, 5320003 (JP)**
• **KIMATA, Ryota**
**Osaka City, 5320003 (JP)**
• **KANEKO, Tsukasa**
**Osaka City, 5320003 (JP)**
• **KAGAWA, Chino**
**Osaka City, 5320003 (JP)**
• **KIMURA, Masataka**
**Osaka City, 5320003 (JP)**

(74) Representative: **Stolmár & Partner**
**Patentanwälte PartG mbB**
**Blumenstraße 17**
**80331 München (DE)**

(54) **SITAGLIPTIN PREPARATION AND METHOD OF STORAGE THEREOF**

(57)    The present invention provides a sitagliptin preparation in which the production of nitrosamines is suppressed. Further, the present invention provides a method of storage of a sitagliptin preparation to suppress the production of nitrosamines. According to an embodiment of the present invention, a sitagliptin preparation is provided comprising a sitagliptin containing tablet, and a case or a packing sealing the sitagliptin containing tablet, wherein an equilibrium relative humidity of the sitagliptin containing tablet as converted at 24°C is 0.3% or less when the sitagliptin preparation is stored for one month under a condition of 25°C and a relative humidity of 60%.

# FIG. 1A

100

**Description**

**TECHNICAL FIELD**

The present invention relates to a sitagliptin preparation. Further, the present invention relates to a method of storage of a sitagliptin preparation.

**BACKGROUND ART**

Sitagliptin phosphate hydrate ((3R)-3-Amino-1-[3-(trifluoromethyl)-5,6-dihydro[1,2,4]triazolo[4,3-α]pyrazin-7(8H)-yl]-4-(2,4,5-trifluorophenyl)butan-1-one monophosphate monohydrate) is an inhibitor of dipeptidyl peptidase-IV (DPP-4) and is used as a therapeutic agent for type II diabetes (for example, Patent Literature 1).

Recently, the European Medicines Agency (EMA) and the U.S. Food and Drug Administration (FDA) have announced that 7-Nitroso-3-(trifluoromethyl)-5,6,7,8-tetrahydro[1,2,4]triazolo-[4,3-a]pyrazine (NTTP), which is classified into nitrosamines, has been detected in a sitagliptin preparation. Nitrosamines are known to be potentially carcinogenic, and an acceptable intake of NTTP is set at 37 ng daily. NTTP is produced by the reaction of 3-(trifluoromethyl)5,6,7,8-tetrahydro[1,2,4]triazolo-[4,3-a]pyrazine, which is produced by hydrolysis of sitagliptin, with nitrogen oxides such as nitrous acid. Nitrogen oxides are contained in small amounts in additives, as well as in the water and air used in the preparation process. Although the production pathway of NTTP is expected as described above, its reactivity is unknown. Therefore, a specific method for reducing the amount of NTTP in a preparation was completely unknown.

**CITATION LIST**

**PATENT LITERATURE**

Patent Literature 1: Japanese Patent No. 3762407

**SUMMARY OF INVENTION**

**TECHNICAL PROBLEM**

Generally, the criterion for reporting the amount of related substances in a preparation is 1,000 ppm, but it is a very difficult task to suppress the production of NTTP in a sitagliptin preparation because a daily acceptable intake of NTTP of 37 ng corresponds to 0.088 ppm.

An object of the present invention is to provide a sitagliptin preparation in which the production of nitrosamines is suppressed. A further object of the present invention is to provide a method of storage of a sitagliptin preparation for suppressing the production of nitrosamines.

**SOLUTION TO PROBLEM**

According to an embodiment of the present invention, a sitagliptin preparation is provided comprising a sitagliptin containing tablet, and a case or a packing sealing the sitagliptin containing tablet, wherein an equilibrium relative humidity of the sitagliptin containing tablet as converted at 24°C is 0.3% or less when the sitagliptin preparation is stored for one month under a condition of 25°C and a relative humidity of 60%.

The case or the packing may provide an airtight condition which can maintain the sitagliptin containing tablet at the equilibrium relative humidity.

The sitagliptin containing tablet may be placed in a blister pack, and the blister pack placed with the sitagliptin containing tablet may be sealed with a pillow package.

The blister pack placing the sitagliptin containing tablet may be sealed with the pillow package along with a desiccant.

The sitagliptin containing tablet may be sealed in an airtight case along with a desiccant.

According to a further embodiment of the present invention, a method of storage of a sitagliptin containing tablet is provided comprising sealing the sitagliptin containing tablet with a case or a packing, wherein an equilibrium relative humidity of the sitagliptin containing tablet as converted at 24°C is 0.3% or less when the sitagliptin preparation is stored for one month under a condition of 25°C and a relative humidity of 60%.

The case or the packing may provide an airtight condition which can maintain the sitagliptin containing tablet at the equilibrium relative humidity.

The sitagliptin containing tablet may be placed in a blister pack, and the blister pack placed with the sitagliptin containing tablet may be sealed with a pillow package.

The blister pack placed with the sitagliptin containing tablet may be sealed with the pillow package along with a desiccant.

The sitagliptin containing tablet may be sealed in an airtight case along with a desiccant.

## ADVANTAGEOUS EFFECT OF INVENTION

According to an embodiment of the present invention, a sitagliptin preparation in which the production of nitrosamines is suppressed is provided. According to a further embodiment of the present invention, a method of storage of a sitagliptin preparation for suppressing the production of nitrosamines is provided.

## BRIEF DESCRIPTION OF DRAWINGS

FIG. 1A is a schematic diagram illustrating a sitagliptin preparation and a method of storage thereof according to an embodiment of the present invention.

FIG. 1B is a schematic diagram illustrating a sitagliptin preparation and a method of storage thereof according to an embodiment of the present invention.

FIG. 2 is a diagram showing a relationship between an equilibrium relative humidity and a natural logarithm of an increasing ratio of NTTP.

## DESCRIPTION OF EMBODIMENTS

Hereinafter, a sitagliptin preparation and a method of storage thereof according to the present invention will be described. Note that the sitagliptin preparation and the method of storage thereof of the present invention are not to be construed as being limited to the description of the following embodiments and examples. In addition, in the present embodiment and the examples to be described later, repetitive descriptions of the same configuration or a configuration having similar functions will be omitted.

In order to satisfy the daily acceptable intake of 37 ng of NTTP, NTTP in a sitagliptin containing tablet at the time of administration should be 0.088 ppm or less. Considering that the reporting threshold for the amount of related substances in a preparation is generally 1,000 ppm, the daily acceptable intake of NTTP is a very stringent criterion. Since the shelf-life of the sitagliptin containing tablet is set at 3 years at room temperature, it is necessary to satisfy the above-mentioned acceptable daily intake at 3 years after manufacture.

As shown in the examples described below, the present inventors have studied and found that a factor of increasing NTTP, which has not been known so far, is correlated with an equilibrium relative humidity of the sitagliptin containing tablet. Furthermore, it was found that in order to satisfy the daily acceptable intake of 37 ng of NTTP, the equilibrium relative humidity of a sitagliptin containing tablet 10 as converted at 24°C was required to be 50.3% or less when a sitagliptin preparation 100 was stored for one month under a condition of 25°C and a relative humidity of 60%. The relationship between the control of the equilibrium relative humidity for suppressing the production of nitrosamines in the sitagliptin containing tablet 10 and the packaging form has not been studied so far, and the fact that the production of nitrosamines in the sitagliptin containing tablet 10 can be suppressed by maintaining the equilibrium relative humidity at 50.3% or less is a surprising effect that could not be predicted from the conventional knowledge.

[Sitagliptin Preparation]

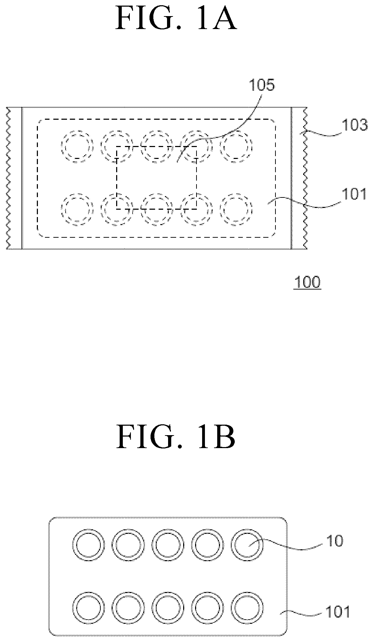

FIG. 1A and FIG. 1B are schematic diagrams illustrating a sitagliptin preparation and storage methods thereof according to an embodiment. The sitagliptin preparation 100 according to the present embodiment includes the sitagliptin containing tablet 10 and a case or a package sealing the sitagliptin containing tablet 10. The equilibrium relative humidity of the sitagliptin containing tablet 10 as converted at 24°C is 50.3% or less when the sitagliptin preparation 100 is stored for one month under a condition of 25°C and a relative humidity of 60%. In the present specification, the term "equilibrium relative humidity" refers to the relative humidity in the absence of moisture exchange between hygroscopic materials and the surrounding environment. In the present specification, the average value of the measured equilibrium relative humidities is converted into the equilibrium relative humidity at 24°C using the average value of measured temperatures and the following equation.

T: Average value of the temperatures during measurement
E: Average value of the measured equilibrium relative humidities
Ps: Saturated water vapor pressure at the average value of the measured temperatures
P: Water vapor pressure at the average value of the measured temperatures
H: Absolute humidity at the average value of the measured temperatures

$$Ps[kPa] = 0.611*10^{\wedge}(7.5*T/(T+237.3))$$

$$P[kPa] = E/100*Ps$$

$$H[kg/kgDA] = 0.62*P/(101.3-P)$$

Equilibrium relative humidity at 24°C [%RH] = H*101.3/(H+0.62)*2.98

In an embodiment, the case or package provides an airtight condition which can maintain the sitagliptin containing tablet 10 at the equilibrium relative humidity. In the present specification, the term "airtight condition" refers to a condition in which a solid or liquid foreign substance does not enter, and loss, efflorescence, deliquescence, or evaporation of a content drug can be prevented. In addition, the airtight condition may be a sealed condition. In the present specification, the "sealed condition" refers to a condition in which gas does not enter.

In FIG. 1A and FIG. 1B, the sitagliptin containing tablet 10 is placed in a blister pack 101 as an embodiment. In FIG. 1A and FIG. 1B, a PTP packaging sheet is shown as an example of the blister pack 101. The blister pack 101 containing the sitagliptin containing tablet 10 is further sealed with a pillow package 103.

The blister pack 101 and the pillow package 103 may be a known blister pack and pillow package, respectively. For example, the blister pack 101 may be a composite sheet of a film selected from a polyvinyl chloride film, a polyvinylidene chloride film, a polychlorotrifluoroethylene film, and a polypropylene. In addition, the blister pack 101 may be a PTP packaging sheet in which an opening of the case made of the above-described films is sealed with aluminum foil.

The pillow package 103 may be an aluminum pillow. Since the pillow package 103 has a packaging form with excellent sealability, the moisture content inside the pillow package 103 can be maintained substantially constant during the storage period. Therefore, when the blister pack 101 is sealed with the pillow package 103 (initial), the equilibrium relative humidity as converted at 24°C in the space inside the pillow package 103 at the time of reaching the steady state is 50.3% or less, preferably 50% or less, more preferably 40% or less, still more preferably 30% or less, and most preferably 20% or less. The lower limit of the equilibrium relative humidity as converted at 24°C is not particularly limited as long as it falls within the above range but is 10% or more, for example,.

In the present specification, the equilibrium relative humidity of the sitagliptin containing tablet as converted at 24°C is evaluated as the equilibrium relative humidity when the sitagliptin preparation 100 is stored for one month under the condition of 25°C and the relative humidity of 60%.

In an embodiment, the blister pack 101 containing the sitagliptin containing tablet 10 may be sealed with the pillow package 103 along with a desiccant 105. The desiccant 105 can absorb the moisture of the sitagliptin containing tablet 10 when the moisture content of the sitagliptin containing tablet 10 is high or when the humidity in the blister pack 101 is high to maintain the equilibrium relative humidity as converted at 24°C in the space inside the pillow package 103 within the above range. In the present embodiment, by sealing the blister pack 101 or by sealing the blister pack 101 along with the desiccant 105, the equilibrium relative humidity as converted at 24°C in the space inside the pillow package 103 that has reached the steady state can be maintained within the above range. As a result, the equilibrium relative humidity as converted at 24°C in the space inside the blister pack 101 that has reached the steady state can also be maintained within the above range, and the equilibrium relative humidity of the sitagliptin containing tablet 10 that has reached the steady state as converted at 24°C can be maintained within the above range.

A known desiccant used as a desiccant for pharmaceuticals can be used as the desiccant 105. It can be selected from, for example, calcium chloride (for example, I.D. (registered trademark) Sheet from I.D. CO., LTD.), zeolite (for example, MS-Serum-W 3G from Tokai Chemical Industry Co., Ltd.), and oxygen scavenger (for example, PharmaKeep (registered trademark) from MITSUBISHI GAS CHEMICAL COMPANY, INC.).

In the case where the desiccant 105 is encapsulated in the pillow package 103, the equilibrium relative humidity is determined by the hygroscopicity of the desiccant 105. In the case where the desiccant 105 is not used, the relative humidity inside the pillow package 103 is determined by the environmental humidity in the controlled working environment for the pillow package.

Furthermore, in an embodiment, the case sealing the sitagliptin containing tablet 10 may be an airtight case such as a tablet bottle or a plastic bottle made of polypropylene or high-density polyethylene. In the present specification, the term "airtight case" refers to a case that cannot allow a solid or liquid foreign substance to enter and can prevent the loss, efflorescence, deliquescence, or evaporation of a content drug, under normal handling, transportation, or storage conditions as defined in the Japanese Pharmacopoeia Eighteenth Edition. In addition, a sealed vessel can be used as the airtight case. In the present specification, the "sealed vessel" refers to a case that does not allow a gas to enter under normal handling, transportation, or storage conditions.

Furthermore, in another embodiment, the sitagliptin containing tablet 10 may be sealed in the case described above along with the desiccant 105.

In another embodiment, the sitagliptin preparation 100 may be contained in, for example, a paper box, and the paper box may be further covered with a vinyl film.

[Sitagliptin containing tablet]

In the present embodiment, the sitagliptin containing tablet 10 may be a known sitagliptin containing tablet. The sitagliptin containing tablet 10 may contain sitagliptin in the form of a pharmaceutically acceptable salt. "Pharmaceutically acceptable salt" refers to salt prepared from non-toxic bases or acids, containing inorganic or organic bases and inorganic or organic acids. Salt obtained from the inorganic base includes aluminum salt, ammonium salt, calcium salt, copper salt, iron (III) salt, iron (II) salt, lithium salt, magnesium salt, manganese (III) salt, manganese (II) salt, potassium salt, sodium salt, and zinc salt. In addition, salt in solid form may exist in more than two crystal structures, and may exist in the form of hydrates. Salts derived from pharmaceutically acceptable non-toxic organic bases include primary amines, secondary amines, tertiary amines, substituted amines including natural substituted amines, cyclic amines, and basic ion-exchange resins such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethyl-piperidine, glucamine, glucosamine, histidine, hydrabamine, isopropylamine, lysine, methylglucamine, morpholine, piperazine, piperidine, polyamine resins, procaine, purine, theobromine, triethylamine, trimethylamine, tripropylamine, and tromethamine.

In addition, the "pharmaceutically acceptable salt" may be salt derived from a pharmaceutically acceptable non-toxic acid, including inorganic acids and organic acids. Such acids include acetic acid, benzenesulfonic acid, benzoic acid, camphorsulfonic acid, citric acid, ethane sulfonic acid, fumaric acid, gluconic acid, glutamic acid, hydrobromic acid, hydrochloric acid, isethionic acid, lactic acid, maleic acid, malic acid, mandelic acid, methanesulfonic acid, mucic acid, nitric acid, pamoic acid, pantothenic acid, phosphoric acid, succinic acid, sulfuric acid, tartaric acid and p-toluenesulfonic acid.

The quantity of sitagliptin contained per tablet of the sitagliptin containing tablet 10 can be set arbitrarily within the range in which a therapeutic effect is obtained, and may be, for example, 12.5 mg, 25 mg, 50 mg, or 100 mg as a free form, and is not particularly limited.

[Method of Storage of Tablet Containing Sitagliptin]

Refer to FIG. 1A and FIG. 1B. In the method of storage of the sitagliptin containing tablet 10, the sitagliptin containing tablet 10 is sealed in a case or package and stored. In the sitagliptin containing tablet 10 stored and sealed in a case or package for one month under a condition of 25°C and a relative humidity of 60%, the equilibrium relative humidity as converted at 24°C is 50.3% or less.

In another embodiment, the case or package provides an airtight condition which can maintain the sitagliptin containing tablet 10 at the equilibrium relative humidity. Furthermore, in another embodiment, the sitagliptin containing tablet 10 may be maintained in a sealed condition by a case or package. In an embodiment, in order to provide the airtight condition, the sitagliptin containing tablet 10 is contained in the blister pack 101, and the blister pack 101 containing the sitagliptin containing tablet 10 may be further sealed with the pillow package 103. Since the details of the blister pack 101 and the pillow package 103 have been described above, a repetitive description thereof is omitted.

Since the pillow package 103 has a packaging form with excellent sealability, the moisture content inside the pillow package 103 can be maintained substantially constant during the storage period. Therefore, when the blister pack 101 is sealed with the pillow package 103 (initial), the equilibrium relative humidity as converted at 24°C in the space inside the pillow package 103 at the time of reaching the steady state is 50.3% or less, preferably 50% or less, more preferably 40% or less, still more preferably 30% or less, and most preferably 20% or less. The lower limit of the equilibrium relative humidity as converted at 24°C is not particularly limited as long as it falls within the above range but is, for example, 10% or more.

The amount of moisture in the space inside the pillow package 103 at the time when the blister pack 101 is sealed with the pillow package 103 (initial) is affected by the amount of moisture in the sitagliptin containing tablet 10 and the humidity of the working environment in which the blister pack 101 is sealed with the pillow package 103. Therefore, in order to ensure that the equilibrium relative humidity as converted at 24°C in the space inside the pillow package 103 that has reached the steady state is 50.3% or less, preferably 50% or less, more preferably 40% or less, still more preferably 30% or less, and most preferably 20% or less when the PTP packaging sheet 101 is sealed with the pillow package 103, the moisture content of the sitagliptin containing tablet 10 itself and the humidity of the working environment in which the PTP packaging sheet 101 is sealed with the pillow package 103 need to be adjusted. On the other hand, since a drying step is generally included in a manufacturing process of the sitagliptin containing tablet 10, the moisture content of the sitagliptin containing tablet 10 itself is adjusted so that the equilibrium relative humidity as converted at 24°C falls within the above range as long as sufficient drying is performed in the manufacturing process.

In an embodiment, the blister pack 101 containing the sitagliptin containing tablet 10 may be sealed with the pillow package 103 along with the desiccant 105. The desiccant 105 can absorb the moisture of the sitagliptin containing tablet 10 when the moisture content of the sitagliptin containing tablet 10 is high or when the humidity in the blister pack 101 is high to maintain the equilibrium relative humidity as converted at 24°C in the space inside the pillow package 103 within the above range. In the present embodiment, by sealing the blister pack 101 or by sealing the blister pack 101 along with the desiccant 105, the equilibrium relative humidity as converted at 24°C in the space inside the pillow package 103 that has reached the steady state can be maintained within the above range. As a result, the equilibrium relative humidity as converted at 24°C in the space inside the blister pack 101 that has reached the steady state can also be maintained within the above range, and the equilibrium relative humidity as converted at 24°C of the sitagliptin containing tablet 10 that has reached the steady state can be maintained within the above range. In addition, since the details of the desiccant 105 have been described above, a repetitive description thereof is omitted.

In the case where the desiccant 105 is encapsulated in the pillow package 103, the equilibrium relative humidity is determined by the hygroscopicity of the desiccant 105. In the case where the desiccant 105 is not used, the relative humidity inside the pillow package 103 is determined by the environmental humidity in the controlled working environment for the pillow package.

Furthermore, in an embodiment, the case sealing the sitagliptin containing tablet 10 may be an airtight case such as a tablet bottle or a plastic bottle made of polypropylene or high-density polyester. Since the details of the airtight case have been described above, a repetitive description thereof is omitted.

In addition, in an embodiment, the sitagliptin containing tablet 10 may be sealed in the case described above along with the desiccant 105.

In another embodiment, the sitagliptin preparation 100 may be contained in, for example, a paper box, and the paper box may be further covered with a vinyl film.

By storing the sitagliptin containing tablet 10 in this manner, the production of NTTP in the sitagliptin containing tablet 10 can be suppressed to a daily acceptable intake of 37 ng.

[EXAMPLES]

[Comparative Examples 1 and 2]

NTTP contained in commercially available sitagliptin containing tablet were evaluated. A commercially available JANUVIA (registered trademark) (50 mg) tablets stored at room temperature for 3 years from production without humidity control was used as Comparative Example 1. In addition, a commercially available JANUVIA (registered trademark) (50 mg) tablets stored at room temperature for one year from production without humidity control was used as Comparative Example 2. Furthermore, the sitagliptin containing tablets of Comparative Examples 1 and 2 were sealed with only the PTP packaging sheet.

[Quantification of NTTP]

2.5 mg of NTTP was weighed and dissolved in a mixture of diluted phosphoric acid (JIS K9005 phosphoric acid special grade 1 ml was diluted with water to 1000 ml) / acetonitrile (19:1) to make exactly 25 ml. 1 ml of this solution was accurately weighed, and the mixture of diluted phosphoric acid (JIS K9005 phosphoric acid special grade 1 ml was diluted with water to 1000 ml) / acetonitrile (19:1) was added to make exactly 100 ml. 1 ml, 2 ml, and 5 ml of this solution were accurately measured, and the mixture of diluted phosphoric acid (JIS K9005 phosphoric acid special grade 1 ml was diluted with water to 1000 ml) / acetonitrile (19:1) was added to make exactly 50 ml, and these solutions were used as a standard solution (4), a standard solution (5), and a standard stock solution, respectively. In addition, 1 ml, 2 ml, and 5 ml of the standard stock solution were accurately measured, and the mixture of diluted phosphoric acid (JIS K9005 phosphoric acid special grade 1 ml was diluted with water to 1000 ml) / acetonitrile (19:1) was added to make exactly 50 ml, and these solutions were used as a standard solution (1), a standard solution (2), and a standard solution (3). 20 μl of each standard solution was taken accurately and measured by a high-performance liquid chromatograph mass spectrometer. The peak area of each standard solution was calculated by an automatic analysis method, and a calibration curve was created from the peak area of each standard solution, with the vertical axis as the peak area and the horizontal axis as the concentration (mg/ml) of the standard solution.

The mass of four sitagliptin containing tablets of Comparative Examples 1 or 2 was precisely measured. 40 ml of the mixture of diluted phosphoric acid (JIS K9005 phosphoric acid special grade 1 ml was diluted with water to 1000 ml) / acetonitrile (19:1) was added, and the tablets were ultrasonicated with occasional shaking until they were completely disintegrated. The mixture of diluted phosphoric acid and acetonitrile (19:1) was further added to make exactly 50 ml. This solution was filtered through a membrane filter having a pore size of 0.2 μm, the first filtrate 2 ml was removed, and the remaining filtrate was used as a sample solution. 20 μl of each sample solution was accurately taken and measured by a high-performance liquid chromatographic mass spectrometer, and the peak area of the sample solution was calculated by the automatic analysis method. The amount of NTTP in the sample solution was obtained from the peak area (Ar) of NTTP of the sample solution using the following relational expression of the amount of NTTP calculated from the calibration curve.

$$\text{Quantity of NTTP (ppm)} = (Ar-b)/a*1/Mr*50000$$

a: Slope of the calibration curve
b: Y-intercept of the calibration curve
Mr: Amount (g) of four tablets of the sample
50000: Correction factor

The amounts of NTTP contained in the sitagliptin containing tablet of Comparative Examples 1 and 2 are shown in Table 1.

[Table 1]

|  | NTTP(ppm) |
|---|---|
| Comparative Example 1 | 0.49 |
| Comparative Example 2 | 0.23 |

The results of Table 1 revealed that the sitagliptin containing tablet of Comparative Example 1 or 2 contained NTTP that significantly exceeded 0.088 ppm of the daily acceptable intake.

[Reference Example 1 and Examples 1 to 3]

The sitagliptin (50 mg) containing tablets in a plastic bottle made of high-density polyethylene along with the desiccant (however, the packing of the bottle was not tightly sealed and not sufficiently airtight) was prepared as Reference Example 1, the sitagliptin (50 mg) containing tablets 50 mg in a plastic bottle made of high-density polyethylene along with the desiccant was prepared as Example 1, the sitagliptin (25 mg) containing tablets placed in a PTP packaging sheet and further sealed with the pillow package along with the desiccant was prepared as Example 2, and the sitagliptin (25 mg) containing tablets in a plastic bottle made of high-density polyethylene along with a desiccant was prepared as Example 3, and they were stored at room temperature or in a cool place for 36 months. The desiccants of Example 1 and Example 3 were different in size.

[Measurement of Equilibrium relative humidity]

The equilibrium relative humidities of the sitagliptin containing tablets of Comparative Examples 1 and 2 were measured using the Moisture Determination Device (Hygrolab2) manufactured by rotronic. 20 tablets of the sitagliptin (50 mg) containing tablets of Reference Example 1 or Example 1 stored under the above conditions, or 30 tablets of the sitagliptin (25 mg) containing tablets of Example 2 or Example 3 were placed in a sample cup, set in a measurement chamber, and measured at room temperature. The equilibrium relative humidity and temperature were recorded when the equilibrium relative humidity was equilibrated. In addition, the equilibrium relative humidity was measured twice for each of the sitagliptin containing tablets of Reference Example 1 and Examples 1 to 3. The average values of the measured equilibrium relative humidity and temperature were converted into the equilibrium relative humidity at 24°C using the following equation.

T: Average value of the temperature during measurement
E: Average value of the measured equilibrium relative humidity
Ps: Saturated water vapor pressure at the average value of the measured temperatures
P: Water vapor pressure at the average value of the measured temperatures
H: Absolute humidity at the average value of the measured temperature

$$Ps[kPa] = 0.611*10^{(7.5*T/(T+237.3))}$$

$$P[kPa] = E/100*Ps$$

$$H[kg/kgDA] = 0.62*P/(101.3-P)$$

Equilibrium relative humidity at 24°C [%RH] = H*101.3/(H+0.62)*2.98

[Quantification of NTTP]

NTTP contained in the sitagliptin containing tablets of Reference Example 1 and Examples 1 to 3 after storage was quantified by the above-described measurement method. NTTP was quantified three times, and the maxima were defined as the amounts of NTTP contained in each tablet.

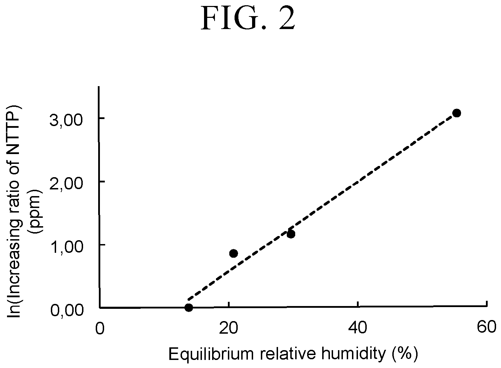

The equilibrium relative humidities and the amounts of NTTP of the sitagliptin containing tablets of Reference Example 1 and Examples 1 to 3 after storage are shown in Table 2. In addition, the relationship between the equilibrium relative humidity and the natural logarithm of the increasing ratio of NTTP is shown in FIG. 2.

[Table 2]

| | Equilibrium relative Humidity (%) | (A) NTTP content (ppm) after 36 months of storage in a cool place | (B) NTTP content (ppm) after 36 months storage at room temperature | ln((B)/(A)) |
|---|---|---|---|---|
| Reference Example 1 | 55.5 | | 0.127 | 3.05 |
| Example 1 | 29.7 | 0.006 | 0.019 | 1.15 |
| Example 2 | 20.8 | | 0.014 | 0.85 |
| Example 3 | 13.8 | | 0.006 | 0.00 |

As shown in FIG. 2, it was found that the amount of NTTP in the sitagliptin containing tablet increased exponentially with respect to the equilibrium relative humidity. The regression equation obtained in FIG. 2 revealed that, when the amount of NTTP at Initial is 0.006 ppm, the equilibrium relative humidity as converted at 24°C at which the amount of NTTP after 36 months becomes 37 ng/ day (0.088 ppm) is 50.3%.

**REFERENCES SIGNS LIST**

10: sitagliptin containing tablet, 100: sitagliptin preparation, 101: packaging sheet, 101: blister pack, 103: pillow package, 105: desiccant

**Claims**

1. A sitagliptin preparation comprising: a sitagliptin containing tablet; and a case or a packing sealing the sitagliptin containing tablet,
   wherein an equilibrium relative humidity of the sitagliptin containing tablet as converted at 24°C is 0.3% or less when the sitagliptin preparation is stored for one month under a condition of 25°C and a relative humidity of 60%.

2. The sitagliptin preparation according to claim 1, wherein the case or the packing provides an airtight condition which can maintain the sitagliptin containing tablet at the equilibrium relative humidity.

3. The sitagliptin preparation according to claim 1,

   wherein the sitagliptin containing tablet is placed in a blister pack, and
   the blister pack placed with the sitagliptin containing tablet is sealed with a pillow package.

4. The sitagliptin preparation according to claim 3,
   wherein the blister pack placed with the sitagliptin containing tablet is sealed with the pillow package along with a desiccant.

5. The sitagliptin preparation according to claim 1,
   wherein the sitagliptin containing tablet is sealed in an airtight case along with a desiccant.

6. A method of storage of a sitagliptin containing tablet comprising:

   sealing the sitagliptin containing tablet with a case or a packing,
   wherein an equilibrium relative humidity of the sitagliptin containing tablet as converted at 24°C is 0.3% or less when the sitagliptin preparation is stored for one month under a condition of 25°C and a relative humidity of 60%.

7. The method of storage of a sitagliptin containing tablet according to claim 6, wherein the case or the packing provides an airtight condition which can maintain the sitagliptin containing tablet at the equilibrium relative humidity.

8. The method of storage of a sitagliptin containing tablet according to claim 6,

   wherein the sitagliptin containing tablet is placed in a blister pack, and
   the blister pack placed with the sitagliptin containing tablet is sealed with a pillow package.

9. The method of storage of a sitagliptin containing tablet according to claim 8,
   wherein the blister pack placed with the sitagliptin containing tablet is sealed with the pillow package along with a desiccant.

10. The method of storage of a sitagliptin containing tablet according to claim 6,
    wherein the sitagliptin containing tablet is sealed in an airtight case along with a desiccant.

# FIG. 1A

105

103

101

100

# FIG. 1B

10

101

FIG. 2

Europäisches
Patentamt

European
Patent Office

Office européen
des brevets

# EUROPEAN SEARCH REPORT

Application Number

EP 24 21 0068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2023 083990 A (TOWA YAKUHIN KK) 16 June 2023 (2023-06-16) * paragraph [0001] * * paragraph [0011] * * paragraph [0041] * * example 1 * * the whole document * | 1-10 | INV. A61K9/20 A61K31/4985 |
| Y | WO 2012/131005 A1 (KRKA TOVARNA ZDRAVIL D D NOVO MESTO [SI]; PRESKAR MAJA [SI] ET AL.) 4 October 2012 (2012-10-04) * page 1, line 4 - line 6 * * page 14, line 18 - line 33 * * page 15, line 1 - line 6 * * the whole document * | 1-10 | |
| Y | WO 2015/114152 A1 (GALENICUM HEALTH SL [ES]) 6 August 2015 (2015-08-06) * page 1, line 2 - line 4 * * page 14 * * the whole document * | 1-10 | TECHNICAL FIELDS SEARCHED (IPC) |
| Y | WO 2014/065427 A1 (SANWA KAGAKU KENKYUSHO CO [JP]; KOWA CO [JP]) 1 May 2014 (2014-05-01) * the whole document * | 1-10 | A61K |

The present search report has been drawn up for all claims

| Place of search | Date of completion of the search | Examiner |
|---|---|---|
| Munich | 28 February 2025 | Felder, Christian |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 21 0068

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2025

| Patent document cited in search report | | Publication date | Patent family member(s) | | Publication date |
|---|---|---|---|---|---|
| JP 2023083990 | A | 16-06-2023 | NONE | | |
| WO 2012131005 | A1 | 04-10-2012 | EA | 201391371 A1 | 28-02-2014 |
| | | | EP | 2691083 A1 | 05-02-2014 |
| | | | SI | 2691083 T1 | 29-12-2017 |
| | | | WO | 2012131005 A1 | 04-10-2012 |
| WO 2015114152 | A1 | 06-08-2015 | CY | 1123022 T1 | 29-10-2021 |
| | | | EP | 3102187 A1 | 14-12-2016 |
| | | | ES | 2792506 T3 | 11-11-2020 |
| | | | HU | E049298 T2 | 28-09-2020 |
| | | | PL | 3102187 T3 | 19-10-2020 |
| | | | PT | 3102187 T | 17-06-2020 |
| | | | SI | 3102187 T1 | 31-08-2020 |
| | | | WO | 2015114152 A1 | 06-08-2015 |
| WO 2014065427 | A1 | 01-05-2014 | JP | 6283316 B2 | 21-02-2018 |
| | | | JP | 6533317 B2 | 19-06-2019 |
| | | | JP | 2018076372 A | 17-05-2018 |
| | | | JP | WO2014065427 A1 | 08-09-2016 |
| | | | WO | 2014065427 A1 | 01-05-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3762407 B

**Non-patent literature cited in the description**

- Japanese Pharmacopoeia